# FASCICULE DE BREVET EUROPEEN

(11) **EP 3 322 364 B1**
(45) Date de publication et mention de la délivrance du brevet: **09.09.2020**
(21) Numéro de dépôt: 16750945.4
(22) Date de dépôt: 11.07.2016
(51) Int. Cl.: A61B 17/88, A61B 17/00

(54) **MANCHE D'ANCILLAIRE ET ENSEMBLE D'INSTRUMENTATION CHIRURGICALE COMPRENANT UN TEL MANCHE D'ANCILLAIRE**
ZUSÄTZLICHER GRIFF UND CHIRURGISCHES INSTRUMENTENSET MIT SOLCH EINEM ZUSÄTZLICHEN GRIFF
ANCILLARY HANDLE AND SURGICAL INSTRUMENTATION SET COMPRISING SUCH AN ANCILLARY HANDLE

(30) Priorité: 16.07.2015 FR 1556720
(43) Date de publication de la demande: 23.05.2018
(73) Titulaire: Novastep, 35760 St Gregoire (FR)
(72) Inventeur: LE BESQUE, Rémi, 35170 Bruz (FR); GIROD, Loïc, 35580 Goven (FR); DULIN, Wilfried, 35320 Crevin (FR)
(74) Mandataire: Chevalier, Renaud Philippe
(86) Numéro de dépôt international: PCT/FR2016/051780
(87) Numéro de publication internationale: WO 2017/009570

(56) Documents cités:
- DE-A1-102004 012 417
- US-A1- 2012 130 388
- US-A1- 2013 096 568

## Description

La présente invention concerne un manche d'ancillaire pour manipuler un instrument chirurgical. De plus, la présente invention concerne un ensemble d'instrumentation chirurgicale comprenant un tel manche d'ancillaire.

Il est connu de l'état de la technique un ancillaire composé d'un manche d'ancillaire et d'un instrument chirurgical fixé au manche d'ancillaire. Le manche d'ancillaire comprend une partie de préhension et une partie de réception ayant un logement allongé configuré pour loger la tige de l'instrument chirurgical. Le manche d'ancillaire comprend en outre un dispositif de verrouillage configuré pour verrouiller rapidement la tige dans le logement allongé de façon à fixer l'instrument chirurgical au manche d'ancillaire. Le dispositif de verrouillage comprend :
- des billes pénétrant dans la gorge,
- des ressorts pour pousser les billes vers la gorge,
- une bague annulaire pour guider les billes, et
- un organe d'actionnement pour déplacer la bague annulaire.

Cependant, un tel dispositif de verrouillage nécessite des pièces nombreuses et petites, ce qui rend le manche d'ancillaire coûteux, complexe et fragile.

Le document DE 10 2004 012 417 A1 divulgue un manche d'ancillaire selon le préambule de la revendication 1.

La présente invention a notamment pour but de résoudre, en tout ou partie, les problèmes mentionnés ci-avant.

Dans ce but, la présente invention a pour objet un manche d'ancillaire selon la revendication 1, pour manipuler un instrument chirurgical comportant une tige, le manche d'ancillaire comprenant :
- une partie de préhension configurée pour permettre à un opérateur de manipuler le manche d'ancillaire,
- une partie de réception ayant un logement allongé configuré pour loger au moins partiellement la tige, et
- un dispositif de verrouillage configuré pour verrouiller rapidement la tige dans le logement allongé de façon à fixer l'instrument chirurgical au manche d'ancillaire ;
le manche d'ancillaire étant caractérisé en ce que le dispositif de verrouillage comprend une partie de verrouillage configurée pour pénétrer dans une gorge située sur la surface externe de la tige, la partie de verrouillage étant déplaçable selon une direction de translation par rapport à la partie de réception et entre :
i) une configuration de verrouillage, dans laquelle la partie de verrouillage peut pénétrer dans la gorge, de sorte que le dispositif de verrouillage peut bloquer l'instrument chirurgical dans le logement allongé, et
ii) une configuration de déverrouillage, dans laquelle la partie de verrouillage peut sortir de la gorge, de sorte que la partie de verrouillage peut libérer la tige,
et en ce que le dispositif de verrouillage comprend en outre une partie d'actionnement agencée pour déplacer la partie de verrouillage entre la configuration de verrouillage et la configuration de déverrouillage, la partie d'actionnement étant solidaire de la partie de verrouillage.

Ainsi, un tel manche d'ancillaire est économique à fabriquer et fiable, car il n'a pas de billes ou autre pièce mobile pour opérer le verrouillage de l'instrument chirurgical. Un tel manche d'ancillaire permet de former un ancillaire après avoir fixé rapidement l'instrument chirurgical au manche d'ancillaire. Le manche d'ancillaire permet à un chirurgien de manipuler l'instrument chirurgical.

Lorsque la partie de verrouillage a pénétré dans la gorge de la tige, le manche d'ancillaire retient l'instrument chirurgical. En particulier, le manche d'ancillaire permet de fixer des instruments chirurgicaux standardisés ayant une connexion conventionnelle de type « AO ». La connexion conventionnelle de type « AO » est parfois désignée sous le terme anglais « AO quick coupling », du nom de la Fondation AO (en allemand : « Arbeitsgemeinschaft für Osteosynthesefragen »).

Selon une variante, la direction de translation est rectiligne.

Selon une variante, le dispositif de verrouillage est lié à la partie de réception. La liaison entre le dispositif de verrouillage et la partie de réception est sélectionnée de façon à permettre une translation de la partie de verrouillage. Par exemple, la liaison entre le dispositif de verrouillage et la partie de réception peut être une liaison glissière.

Le dispositif de verrouillage peut bloquer l'instrument chirurgical dans le logement allongé de façon à empêcher une translation de l'instrument chirurgical selon une direction d'allongement du logement allongé.

Selon un mode de réalisation, le logement allongé a une forme comprenant une portion angulaire de cylindre et un méplat, le méplat étant adapté pour coopérer avec un méplat complémentaire situé sur la surface externe de la tige, de façon à empêcher une rotation de la tige dans le logement allongé.

Ainsi, un tel logement allongé permet de loger et de retenir une partie de la tige tout en empêchant la rotation de la tige.

Selon un mode de réalisation, la direction de translation s'étend dans un plan perpendiculaire à la direction d'allongement du logement allongé.

Ainsi, une telle direction de translation permet de former un dispositif de verrouillage peu encombrant selon la direction d'allongement du logement allongé.

Selon un mode de réalisation, la partie de verrouillage a une amplitude de déplacement comprise entre 2 mm et 8 mm.

Ainsi, une telle amplitude de déplacement est minimisée, ce qui permet un verrouillage et un déverrouillage rapide par le chirurgien.

Selon un mode de réalisation, la partie de verrouillage présente une surface partiellement torique de façon à pénétrer dans la gorge ayant forme partiellement torique complémentaire.

Ainsi, une telle surface partiellement torique permet de maximiser l'interface de contact entre la partie de verrouillage et la gorge. Donc le manche d'ancillaire est adapté pour retenir des instruments chirurgicaux standardisés ayant une connexion conventionnelle de type « AO », avec une gorge torique ou partiellement torique.

Selon un mode de réalisation, la surface partiellement torique s'étend sur un secteur angulaire compris entre 40 degrés et 180 degrés, le sommet du secteur angulaire appartenant à l'axe de révolution de la surface partiellement torique.

Ainsi, la surface partiellement torique sur un tel secteur angulaire permet de retenir fermement la tige dans le logement, donc de verrouiller efficacement l'instrument chirurgical dans le manche d'ancillaire. En effet, un tel secteur angulaire confère à la surface partiellement torique une interface de contact étendue avec la gorge de la tige.

Selon une variante, la surface partiellement torique est formée par une portion interne de tore. Selon une variante, la surface partiellement torique a une base en arc de cercle.

Selon un mode de réalisation, la partie d'actionnement et la partie de réception sont situées à une même extrémité de la partie de préhension.

En d'autres termes, la partie d'actionnement est située près de la partie de réception. Ainsi, le manche d'ancillaire est ergonomique, car le chirurgien peut facilement manipuler la partie d'actionnement, contrairement à une partie d'actionnement de l'état de la technique située à l'opposé de la partie de réception, donc de la tige, par rapport à la partie de préhension.

De plus, le manche d'ancillaire offre une grande sécurité de manipulation, car le chirurgien ne risque pas de déverrouiller l'instrument chirurgical inopinément en exerçant une pression sur la partie de préhension ; tandis que l'état de la technique présente parfois une partie d'actionnement située sur une portion de la partie de réception sur laquelle le chirurgien exerce souvent une pression, ce qui risque de déverrouiller inopinément l'instrument chirurgical.

Selon un mode de réalisation, la partie d'actionnement comprend un bouton de verrouillage et un bouton de déverrouillage, le bouton de verrouillage étant agencé pour déplacer la partie de verrouillage vers la configuration de verrouillage, le bouton de déverrouillage étant agencé pour déplacer la partie de verrouillage vers la configuration de déverrouillage.

Ainsi, de tels boutons de verrouillage et de déverrouillage facilitent la manipulation du manche d'ancillaire par un chirurgien, par exemple entre le pouce et l'index.

Selon un mode de réalisation, le bouton de verrouillage et le bouton de déverrouillage sont solidaires et situés sur deux côtés opposés du logement allongé.

En d'autres termes, le bouton de verrouillage et le bouton de déverrouillage sont déplacés de manière symétrique et ils fonctionnent en quelque sorte en opposition de phases. Ainsi, le chirurgien peut manipuler facilement la partie d'actionnement, car les gestes de verrouillage et de déverrouillage sont symétriques.

Selon l'invention présente, le dispositif de verrouillage comporte au moins une partie d'immobilisation, la partie d'immobilisation étant solidaire en translation de la partie de verrouillage, de sorte que la partie d'immobilisation est déplaçable entre :
i) un état d'immobilisation, dans lequel la partie d'immobilisation permet une immobilisation du dispositif de verrouillage par rapport à la partie de réception lorsque la partie de verrouillage est en configuration de verrouillage, et
ii) un état de dégagement, dans lequel la partie d'immobilisation libère le dispositif de verrouillage de la partie de réception lorsque la partie de verrouillage est en configuration de déverrouillage,
la partie d'immobilisation étant configurée pour autoriser un nombre maximal d'immobilisations, de sorte que la partie d'immobilisation ne permet plus une immobilisation lorsque le nombre maximal est atteint.

Ainsi, une telle partie d'immobilisation n'autorise qu'un usage du manche d'ancillaire en nombre limité, donc limité dans le temps, par exemple limité à une opération chirurgicale, ce qui garantit la sécurité du patient, car il n'y aucun risque de réutilisation inopinée du manche d'ancillaire qui ne serait plus stérile.

Selon un mode de réalisation, le nombre maximal est compris entre 5 et 50, par exemple entre 5 et 20.

Ainsi, le manche d'ancillaire est pratiquement à usage unique.

Selon un mode de réalisation, la partie d'immobilisation est configurée pour subir une déformation plastique, par exemple par matage, lors de chaque immobilisation, de sorte que la partie d'immobilisation est usée lorsque le nombre maximal est atteint.

Ainsi, une telle partie d'immobilisation plastiquement déformable permet de limiter les immobilisations au nombre maximal.

Selon un mode de réalisation, la partie de verrouillage a une embase, la partie d'immobilisation comprenant au moins une surépaisseur de matière qui s'étend sur un côté respectif de l'embase.

Ainsi, une telle surépaisseur de matière permet de limiter le nombre d'immobilisation, car la surépaisseur de matière est matée, donc partiellement aplatie, à chaque immobilisation. Les dimensions de la surépaisseur de matière peuvent être sélectionnées en fonction des propriétés mécaniques de la matière (coefficient de frottement, dureté) et de la sensation de verrouillage requise. et la partie de réception sont composées d'un matériau sélectionné dans le groupe constitué des polyarylamides (PAA, par exemple l'IXEF®) et des polyphénylsulfones (PPSA, par exemple le RADEL®).

Selon une variante, la partie d'immobilisation comprend deux surépaisseurs de matière qui s'étendent respectivement sur deux côtés opposés de l'embase. Par exemple, les surépaisseurs de matière peuvent être formées par des godrons. Chaque surépaisseur de matière peut avoir une forme rectiligne s'étendant sensiblement parallèlement à la direction de translation.

Alternativement à cette variante, la partie d'immobilisation comprend une surface conique et la partie de réception a un logement conique complémentaire, la surface conique et le logement conique étant configurés pour opérer un emmanchement de type cône Morse.

Selon un mode de réalisation, la partie de réception a un passage primaire et la partie de préhension a un passage secondaire, le passage primaire et le passage secondaire étant coïncidents de façon à passer une broche de guidage à travers les passages primaire et secondaire.

Ainsi, un chirurgien peut implanter temporairement des broches de guidage, puis enfiler un instrument chirurgical canulé et le manche d'ancillaire sur la broche de guidage, de façon à guider son geste de manière précise.

Par ailleurs, la présente invention a pour objet un ensemble d'instrumentation chirurgicale comprenant :
- un manche d'ancillaire selon l'invention, et
- plusieurs instruments chirurgicaux comportant chacun une tige, la surface externe de chaque tige ayant une gorge adaptée pour recevoir la partie de verrouillage, de sorte que les instruments chirurgicaux sont interchangeables.

Ainsi, un tel ensemble d'instrumentation chirurgicale permet une manipulation aisée par le chirurgien et le manche d'ancillaire est économique et sûr.

Selon un mode de réalisation, la surface externe de la partie de préhension a des rainures longitudinales, chaque rainure longitudinale étant adaptée pour loger la tige d'un instrument chirurgical respectif de façon à fixer ledit instrument chirurgical par encliquetage élastique.

Ainsi, l'ensemble d'instrumentation chirurgicale est compact, ce qui facilite son transport et son stockage.

Les modes de réalisation et les variantes mentionnés ci-avant peuvent être pris isolément ou selon toute combinaison techniquement possible.

La présente invention sera bien comprise et ses avantages ressortiront aussi à la lumière de la description qui va suivre, donnée uniquement à titre d'exemple non limitatif et faite en référence aux figures schématiques annexées, dans lesquelles des signes de références identiques correspondent à des éléments structurellement et/ou fonctionnellement identiques ou similaires. Dans les figures schématiques annexées :
- la figure 1 est une vue de côté d'un manche d'ancillaire conforme à l'invention ;
- les figures 2 et 3 sont des vues en perspective, suivant deux angles opposés, d'une partie du manche d'ancillaire de la figure 1 ;
- la figure 4 est une vue de côté d'un ensemble d'instrumentation chirurgicale conforme à l'invention, en configuration de service et comprenant le manche d'ancillaire de la figure 1 ;
- la figure 5 est une vue de côté d'un instrument chirurgical appartenant à l'ensemble d'instrumentation chirurgicale de la figure 4 ;
- la figure 6 est une vue en perspective de l'ensemble d'instrumentation chirurgicale de la figure 4, en configuration de rangement ;
- la figure 7 est une vue en perspective d'un dispositif de verrouillage appartenant au manche d'ancillaire de la figure 1 ;
- la figure 8 est une vue de face du dispositif de verrouillage de la figure 7 ;
- la figure 9 est une vue à plus grande échelle du détail IX à la figure 8 ;
- la figure 10 est une vue en perspective tronquée d'une partie du dispositif de verrouillage de la figure 1 dans une configuration de verrouillage ;
- la figure 11 est une coupe de la partie illustrée à la figure 10 ;
- la figure 12 est une coupe, selon la ligne XII à la figure 11, dans une configuration de verrouillage ;
- la figure 13 est une coupe, selon la ligne XIII à la figure 11, dans une configuration de déverrouillage ; et
- la figure 14 est une coupe similaire à la figure 13, dans une configuration de verrouillage.

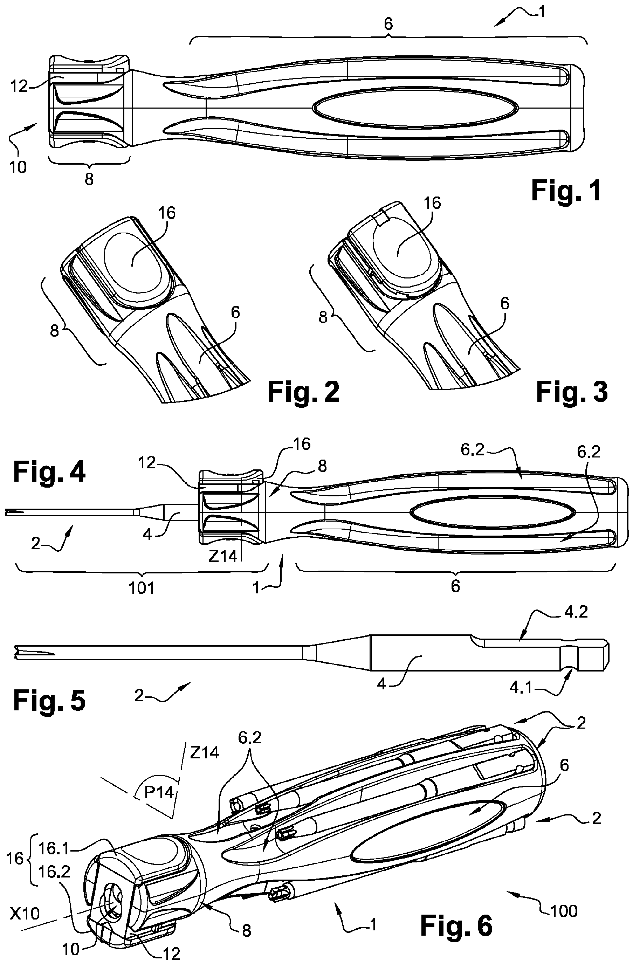

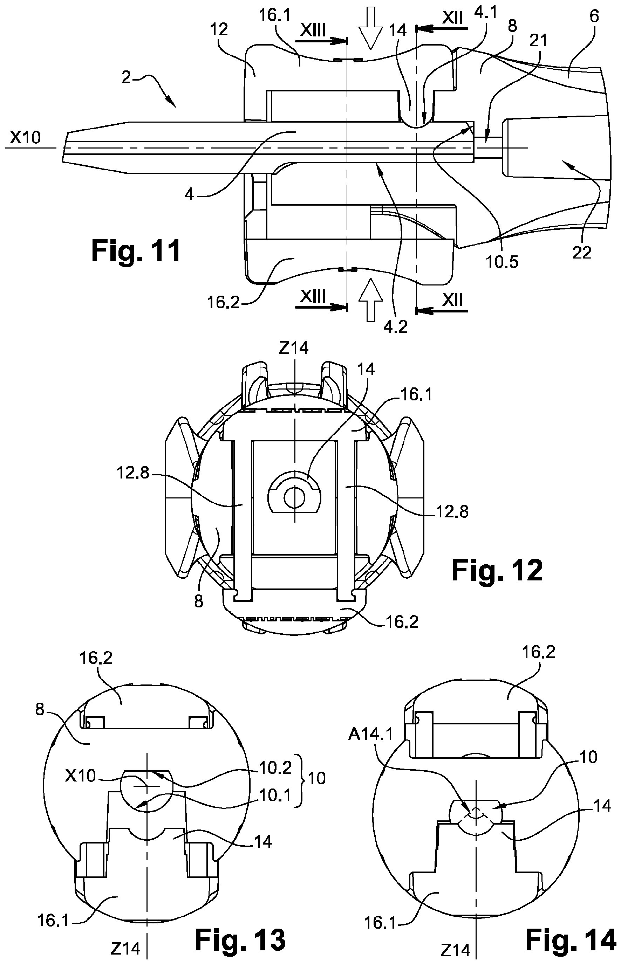

Les figures 1 à 14 illustrent un manche d'ancillaire 1 pour manipuler un instrument chirurgical 2 comportant une tige 4. Le manche d'ancillaire 1 équipé d'un instrument chirurgical 2 forme un ensemble d'instrumentation chirurgicale 100.

Le manche d'ancillaire 1 comprend une partie de préhension 6, une partie de réception 8 et un dispositif de verrouillage 12. La partie de préhension 6 est configurée pour permettre à un opérateur de manipuler le manche d'ancillaire 1. La partie de réception 8 a un logement allongé 10 qui est configuré pour loger au moins partiellement la tige 4. Le dispositif de verrouillage 12 est configuré pour verrouiller rapidement la tige 4 dans le logement allongé 10, de façon à fixer l'instrument chirurgical 2 au manche d'ancillaire 1.

Le dispositif de verrouillage 12 comprend une partie de verrouillage 14 qui est configurée pour pénétrer dans une gorge 4.1 située sur la surface externe de la tige 4. La partie de verrouillage 14 est déplaçable selon une direction de translation Z14 par rapport à la partie de réception 8. La partie de verrouillage 14 est mobile entre :
iii) une configuration de verrouillage (figures 2, 6, 10, 11 et 14), dans laquelle la partie de verrouillage 14 peut pénétrer dans la gorge 4.1, de sorte que le dispositif de verrouillage 12 peut bloquer l'instrument chirurgical 2 dans le logement allongé 10, et
iv) une configuration de déverrouillage (figures 3, 12 et 13), dans laquelle la partie de verrouillage 14 peut sortir de la gorge 4.1, de sorte que la partie de verrouillage 14 peut libérer la tige 4.

La direction de translation Z14 est rectiligne et s'étend dans un plan perpendiculaire P14 à la direction d'allongement X10 du logement allongé 10. La partie de verrouillage 14 a une amplitude de déplacement environ égale à 5 mm parallèlement à la direction de translation Z14.

Le dispositif de verrouillage 12 comprend en outre une partie d'actionnement 16. La partie d'actionnement 16 est agencée pour déplacer la partie de verrouillage 14, selon la direction de translation Z14, entre la configuration de verrouillage (figures 2, 6, 10, 11 et 14) et la configuration de déverrouillage (figures 3, 12 et 13).

Le dispositif de verrouillage 12 est lié à la partie de réception 8. La liaison entre le dispositif de verrouillage 12 et la partie de réception 8 est sélectionnée de façon à permettre une translation de la partie de verrouillage 14. Dans l'exemple des figures, la liaison entre le dispositif de verrouillage 12 et la partie de réception 8 est une liaison glissière ou coulissante 12.8.

La partie d'actionnement 16 est solidaire de la partie de verrouillage 14. La partie d'actionnement 16 et la partie de réception 8 sont ici situées à une même extrémité de la partie de préhension 6, ce qui rend le manche d'ancillaire 1 ergonomique.

La partie de réception 8 a un passage primaire 21 et la partie de préhension 6 a un passage secondaire 22. Le passage primaire 21 et le passage secondaire 22 coïncident de façon à permettre le passage d'une même broche de guidage à travers le passage primaire 21 et le passage secondaire 22.

La partie d'actionnement 16 comprend un bouton de verrouillage 16.1 et un bouton de déverrouillage 16.2. Le bouton de verrouillage 16.1 et le bouton de déverrouillage 16.2 sont ici solidaires et situés sur deux côtés opposés du logement allongé 10.

Le bouton de verrouillage 16.1 est agencé pour déplacer la partie de verrouillage 14 vers la configuration de verrouillage (figures 2, 6, 10, 11 et 14). Le bouton de déverrouillage 16.2 est agencé pour déplacer la partie de verrouillage 14 vers la configuration de déverrouillage (figures 3, 12 et 13).

Le bouton de verrouillage 16.1 et le bouton de déverrouillage 16.2 sont déplacés de manière symétrique et ils fonctionnent en opposition de phases.

Ainsi, de tels boutons de verrouillage 16.1 et de déverrouillage 16.2 facilitent la manipulation du manche d'ancillaire 1 par un chirurgien, entre le pouce et l'index.

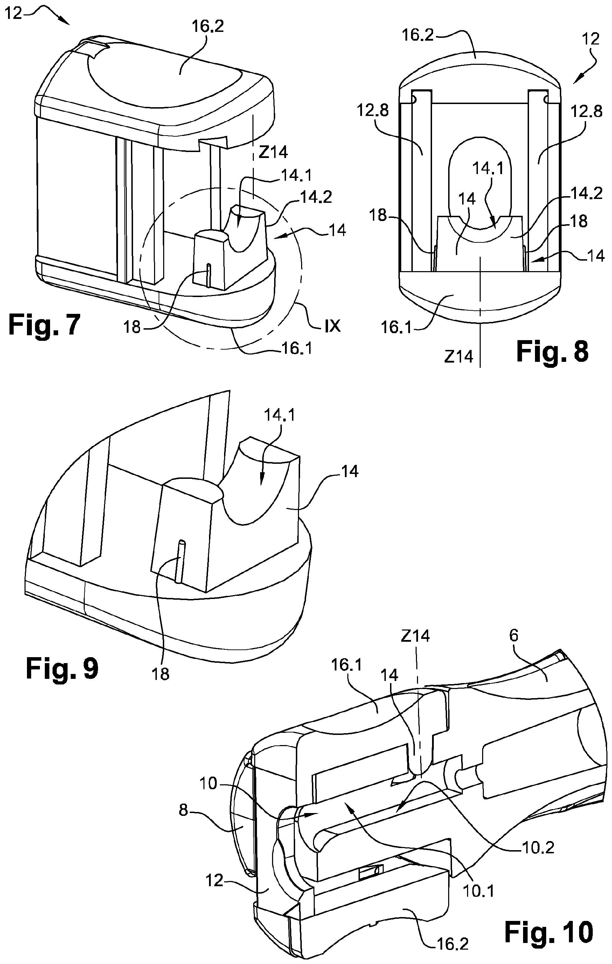

Comme l'illustrent les figures 5 et 10, le logement allongé 10 a une forme comprenant une portion angulaire de cylindre 10.1 et un méplat 10.2. Le méplat 10.2 est adapté pour coopérer avec un méplat complémentaire 4.2 situé sur la surface externe de la tige 4, de façon à empêcher une rotation de la tige 4 dans le logement allongé 10.

De plus, le logement allongé 10 présente une butée mécanique 10.5 qui a pour fonction de positionner l'instrument chirurgical 2 dans le manche d'ancillaire 1 selon la direction longitudinale d'allongement X10.

Ainsi, grâce à la coopération des méplats 4.2 et 10.2, le logement allongé 10 permet de loger et de retenir une partie de la tige 4 tout en empêchant la rotation de la tige 4.

Comme l'illustrent les figures 7, 8 et 9, la partie de verrouillage 14 présente une surface partiellement torique 14.1 qui est configurée pour pénétrer dans la gorge 4.1, laquelle a une forme partiellement torique complémentaire. Dans l'exemple des figures, la surface partiellement torique 14.1 est formée par une portion interne de tore, avec une base en arc de cercle.

Comme le montre la figure 1, la surface partiellement torique 14.1 s'étend sur un secteur angulaire A14.1 environ égal à 120 degrés. Le sommet du secteur angulaire A14.1 appartenant à l'axe de révolution de la surface partiellement torique 14.1. La surface partiellement torique 14.1 est qualifiée de partiellement torique en raison de son extension sur un secteur angulaire inférieur à 360 degrés.

Ainsi, la surface partiellement torique 14.1 permet de maximiser l'interface de contact entre la partie de verrouillage 14 et la gorge 4.1. Donc le manche d'ancillaire 1 est adapté pour retenir des instruments chirurgicaux 2 standardisés ayant une connexion conventionnelle de type « AO ».

Lorsque la partie de verrouillage 14 a pénétré dans la gorge 4.1 de la tige 4, le manche d'ancillaire 1 retient l'instrument chirurgical 2, donc immobilise l'instrument chirurgical 2 en translation selon la direction d'allongement X10.

Par ailleurs, le dispositif de verrouillage 12 comporte une partie d'immobilisation 18 qui est solidaire en translation de la partie de verrouillage 14, de sorte que la partie d'immobilisation 18 est déplaçable entre :
iii) un état d'immobilisation, dans lequel la partie d'immobilisation 18 permet une immobilisation du dispositif de verrouillage 12 par rapport à la partie de réception 8 lorsque la partie de verrouillage 14 est en configuration de verrouillage (figures 2, 6, 10, 11 et 14), et
iv) un état de dégagement, dans lequel la partie d'immobilisation 18 libère le dispositif de verrouillage 12 de la partie de réception 8 lorsque la partie de verrouillage 14 est en configuration de déverrouillage (figures 3, 12 et 13),

La partie d'immobilisation 18 est configurée pour autoriser un nombre maximal d'immobilisations, de sorte que la partie d'immobilisation 18 ne permet plus une immobilisation lorsque le nombre maximal est atteint. Le nombre maximal est ici environ égal à 20.

Ainsi, la partie d'immobilisation 18 n'autorise qu'un usage du manche d'ancillaire 1 qui est limité dans le temps, par exemple limité à une opération chirurgicale.

La partie d'immobilisation 18 est ici configurée pour subir une déformation plastique par matage lors de chaque immobilisation d'un instrument chirurgical 2, de sorte que la partie d'immobilisation 18 est usée lorsque le nombre maximal (ici 20) est atteint.

La partie de verrouillage 14 a une embase 14.2. La partie d'immobilisation 18 comprend deux surépaisseurs de matière qui s'étendent respectivement sur les deux côtés de l'embase 14.2. Les surépaisseurs de matière sont ici formées par des godrons ayant chacun une forme rectiligne s'étendant sensiblement parallèlement à la direction de translation Z14.

Ces surépaisseurs de matière permettent de limiter le nombre d'immobilisation. En effet, chaque surépaisseur de matière est matée, donc partiellement aplatie, à chaque immobilisation.

Par ailleurs, la présente invention a pour objet un ensemble d'instrumentation chirurgicale 100 comprenant le manche d'ancillaire 1 et plusieurs instruments chirurgicaux 2. Chacun des instruments chirurgicaux 2 comporte une tige 4, dont la surface externe a une gorge 4.1. Comme précisé ci-avant, la gorge 4.1 est adaptée pour recevoir la partie de verrouillage 14, de sorte que les instruments chirurgicaux 2 sont interchangeables.

La surface externe de la partie de préhension 6 a des rainures longitudinales 6.2. Chaque rainure longitudinale 6.2 est adaptée pour loger la tige 4 d'un instrument chirurgical respectif 2, de façon à fixer cet instrument chirurgical 2 par encliquetage élastique.

Bien entendu, la présente invention n'est pas limitée aux modes de réalisation particuliers décrits dans la présente demande de brevet, ni à des modes de réalisation à la portée de l'homme du métier. D'autres modes de réalisation peuvent être envisagés sans sortir du cadre de l'invention, à partir de tout élément équivalent à un élément indiqué dans la présente demande de brevet.

## Revendications

1. Manche d'ancillaire (1), pour manipuler un instrument chirurgical (2) comportant une tige (4), le manche d'ancillaire (1) comprenant :
- une partie de préhension (6) configurée pour permettre à un opérateur de manipuler le manche d'ancillaire (1),
- une partie de réception (8) ayant un logement allongé (10) configuré pour loger au moins partiellement la tige (4), et
- un dispositif de verrouillage (12) configuré pour verrouiller rapidement la tige (4) dans le logement allongé (10) de façon à fixer l'instrument chirurgical (2) au manche d'ancillaire (1) ;
le dispositif de verrouillage (12) comprenant une partie de verrouillage (14) configurée pour pénétrer dans une gorge (4.1) située sur la surface externe de la tige (4), la partie de verrouillage (14) étant déplaçable selon une direction de translation (Z14) par rapport à la partie de réception (8) et entre :
i) une configuration de verrouillage, dans laquelle la partie de verrouillage (14) peut pénétrer dans la gorge (4.1), de sorte que le dispositif de verrouillage (12) peut bloquer l'instrument chirurgical (2) dans le logement allongé (10), et
ii) une configuration de déverrouillage, dans laquelle la partie de verrouillage (14) peut sortir de la gorge (4.1), de sorte que la partie de verrouillage (14) peut libérer la tige (4),
le dispositif de verrouillage (12) comprenant en outre une partie d'actionnement (16) agencée pour déplacer la partie de verrouillage (14) entre la configuration de verrouillage et la configuration de déverrouillage, la partie d'actionnement (16) étant solidaire de la partie de verrouillage (14)
le dispositif de verrouillage (12) étant **caractérisé en ce qu'**il comporte au moins une partie d'immobilisation (18), la partie d'immobilisation (18) étant solidaire en translation de la partie de verrouillage (14), de sorte que la partie d'immobilisation (18) est déplaçable entre :
i) un état d'immobilisation, dans lequel la partie d'immobilisation (18) permet une immobilisation du dispositif de verrouillage (12) par rapport à la partie de réception (8) lorsque la partie de verrouillage (14) est en configuration de verrouillage, et
ii) un état de dégagement, dans lequel la partie d'immobilisation (18) libère le dispositif de verrouillage (12) de la partie de réception (8) lorsque la partie de verrouillage (14) est en configuration de déverrouillage,
la partie d'immobilisation (18) étant configurée pour autoriser un nombre maximal d'immobilisations, de sorte que la partie d'immobilisation (18) ne permet plus une immobilisation lorsque le nombre maximal est atteint.

2. Manche d'ancillaire (1) selon la revendication 1, dans lequel le logement allongé (10) a une forme comprenant une portion angulaire de cylindre (10.1) et un méplat (10.2), le méplat (10.2) étant adapté pour coopérer avec un méplat complémentaire (4.2) situé sur la surface externe de la tige (4), de façon à empêcher une rotation de la tige (4) dans le logement allongé (10).

3. Manche d'ancillaire (1) selon l'une quelconque des revendications précédentes, dans lequel la direction de translation (Z14) s'étend dans un plan perpendiculaire (P14) à la direction d'allongement (X10) du logement allongé (10).

4. Manche d'ancillaire (1) selon l'une quelconque des revendications précédentes, dans lequel la partie de verrouillage (14) est configurée pour avoir une amplitude de déplacement comprise entre 2 mm et 8 mm.

5. Manche d'ancillaire (1) selon l'une quelconque des revendications précédentes, dans lequel la partie de verrouillage (14) présente une surface partiellement torique (14.1) de façon à pénétrer dans la gorge (4.1) ayant forme partiellement torique complémentaire.

6. Manche d'ancillaire (1) selon la revendication précédente, dans lequel la surface partiellement torique (14.1) s'étend sur un secteur angulaire (A14.1) compris entre 40 degrés et 180 degrés, le sommet du secteur angulaire (A14.1) appartenant à l'axe de révolution de la surface partiellement torique (14.1).

7. Manche d'ancillaire (1) selon l'une quelconque des revendications précédentes, dans lequel la partie d'actionnement (16) et la partie de réception (8) sont situées à une même extrémité de la partie de préhension (6).

8. Manche d'ancillaire (1) selon l'une quelconque des revendications précédentes, dans lequel la partie d'actionnement (16) comprend un bouton de verrouillage (16.1) et un bouton de déverrouillage (16.2), le bouton de verrouillage (16.1) étant agencé pour déplacer la partie de verrouillage (14) vers la configuration de verrouillage, le bouton de déverrouillage (16.2) étant agencé pour déplacer la partie de verrouillage (14) vers la configuration de déverrouillage.

9. Manche d'ancillaire (1) selon la revendication précédente, dans lequel le bouton de verrouillage (16.1) et le bouton de déverrouillage (16.2) sont solidaires et situés sur deux côtés opposés du logement allongé (10).

10. Manche d'ancillaire (1) selon la revendication précédente, dans lequel le nombre maximal est compris entre 5 et 50, par exemple entre 5 et 20.

11. Manche d'ancillaire (1) selon l'une quelconque des revendications précédentes, dans lequel la partie d'immobilisation (18) est configurée pour subir une déformation plastique, par exemple par matage, lors de chaque immobilisation, de sorte que la partie d'immobilisation (18) est usée lorsque le nombre maximal est atteint.

12. Manche d'ancillaire (1) selon la revendication précédente, dans lequel la partie de verrouillage (14) a une embase (14.2), la partie d'immobilisation (18) comprenant au moins une surépaisseur de matière qui s'étend sur un côté respectif de l'embase (14.2).

13. Manche d'ancillaire (1) selon l'une quelconque des revendications précédentes, dans lequel la partie de réception (8) a un passage primaire (21) et la partie de préhension (6) a un passage secondaire (22), le passage primaire (21) et le passage secondaire (22) étant coïncidents de façon à passer une broche de guidage à travers les passages primaire (21) et secondaire (22).

14. Ensemble d'instrumentation chirurgicale (100) comprenant :
- un manche d'ancillaire (1) selon l'une quelconque des revendications précédentes, et
- plusieurs instruments chirurgicaux (2) comportant chacun une tige (4), la surface externe de chaque tige (4) ayant une gorge (4.1) adaptée pour recevoir la partie de verrouillage (14), de sorte que les instruments chirurgicaux (2) sont interchangeables.

15. Ensemble d'instrumentation chirurgicale (100) selon la revendication précédente, dans lequel la surface externe de la partie de préhension (6) a des rainures longitudinales (6.2), chaque rainure longitudinale (6.2) étant adaptée pour loger la tige (4) d'un instrument chirurgical respectif (2) de façon à fixer ledit instrument chirurgical (2) par encliquetage élastique.

## Patentansprüche

1. Zusätzlicher Griff (1) zur Handhabung eines chirurgischen Instruments (2), aufweisend einen Schaft (4), wobei der zusätzliche Griff (1) umfasst:
- einen Greifteil (6), der ausgelegt ist, um einem Bediener die Handhabung des zusätzlichen Griffs (1) zu erlauben,
- einen Empfangsteil (8) mit einer länglichen Aufnahme (10), die ausgelegt ist, um den Schaft (4) mindestens teilweise aufzunehmen, und
- eine Verriegelungsvorrichtung (12), die ausgelegt ist, um den Schaft (4) in der länglichen Aufnahme (10) schnell zu verriegeln, um das chirurgische Instrument (2) am zusätzlichen Griff (1) zu befestigen;
wobei die Verriegelungsvorrichtung (12) einen Verriegelungsteil (14) umfasst, der ausgelegt ist, um in eine Nut (4.1) einzudringen, die sich auf der Außenfläche des Schafts (4) befindet, wobei der Verriegelungsteil (14) gemäß einer Translationsrichtung (Z14) in Bezug auf den Empfangsteil (8) verlagerbar ist und zwischen:
i) einer Verriegelungskonfiguration, in der der Verriegelungsteil (14) in die Nut (4.1) eindringen kann, so dass die Verriegelungsvorrichtung (12) das chirurgische Instrument (2) in der länglichen Aufnahme (10) arretieren kann, und
ii) einer Entriegelungskonfiguration, in der der Verriegelungsteil (14) die Nut (4.1) verlassen kann, so dass der Verriegelungsteil (14) den Schaft (4) freigeben kann,
wobei die Verriegelungsvorrichtung (12) ferner einen Betätigungsteil (16) umfasst, der eingerichtet ist, um den Verriegelungsteil (14) zwischen der Verriegelungskonfiguration und der Entriegelungskonfiguration zu verlagern, wobei der Betätigungsteil (16) mit dem Verriegelungsteil (14) fest verbunden ist,
wobei die Verriegelungsvorrichtung (12) **dadurch gekennzeichnet ist, dass** sie mindestens einen Arretierungsteil (18) aufweist, wobei der Arretierungsteil (18) mit dem Verriegelungsteil (14) translatorisch fest verbunden ist, so dass der Arretierungsteil (18) verlagerbar ist zwischen:
i) einem Arretierungszustand, in dem der Arretierungsteil (18) eine Arretierung der Verriegelungsvorrichtung (12) in Bezug auf den Empfangsteil (8) erlaubt, wenn der Verriegelungsteil (14) in Verriegelungskonfiguration ist, und
ii) einem Freisetzungszustand, in dem der Arretierungsteil (18) die Verriegelungsvorrichtung (12) vom Empfangsteil (8) freigibt, wenn der Verriegelungsteil (14) in Entriegelungskonfiguration ist,
wobei der Arretierungsteil (18) ausgelegt ist, um eine maximale Anzahl von Arretierungen zu gestatten, so dass der Arretierungsteil (18) keine Arretierung mehr erlaubt, wenn die maximale Anzahl erreicht ist.

2. Zusätzlicher Griff (1) nach Anspruch 1, wobei die längliche Aufnahme (10) eine Form hat, umfassend einen winkligen Zylinderabschnitt (10.1) und eine Abflachung (10.2), wobei die Abflachung (10.2) zum Zusammenwirken mit einer komplementären Abflachung (4.2) geeignet ist, die sich auf der Außenfläche des Schafts (4) befindet, um eine Rotation des Schafts (4) in der länglichen Aufnahme (10) zu verhindern.

3. Zusätzlicher Griff (1) nach einem der vorangehenden Ansprüche, wobei sich die Translationsrichtung (Z14) in einer Ebene (P14) erstreckt, die senkrecht zur Längsrichtung (X10) der länglichen Aufnahme (10) ist.

4. Zusätzlicher Griff (1) nach einem der vorangehenden Ansprüche, wobei der Verriegelungsteil (14) ausgelegt ist, um eine Verlagerungsamplitude zu haben, die zwischen 2 mm und 8 mm liegt.

5. Zusätzlicher Griff (1) nach einem der vorangehenden Ansprüche, wobei der Verriegelungsteil (14) eine teilweise torische Fläche (14.1) zwecks Eindringens in die Nut (4.1) aufweist, die eine komplementäre teilweise torische Form hat.

6. Zusätzlicher Griff (1) nach vorangehendem Anspruch, wobei sich die teilweise torische Fläche (14.1) über einen Winkelsektor (A14.1) erstreckt, der zwischen 40 Grad und 180 Grad liegt, wobei die Spitze des Winkelsektors (A14.1) zu der Drehachse der teilweisen torischen Fläche (14.1) gehört.

7. Zusätzlicher Griff (1) nach einem der vorangehenden Ansprüche, wobei sich der Betätigungsteil (16) und der Empfangsteil (8) an einem selben Ende des Greifteils (6) befinden.

8. Zusätzlicher Griff (1) nach einem der vorangehenden Ansprüche, wobei der Betätigungsteil (16) einen Verriegelungsknopf (16.1) und einen Entriegelungsknopf (16.2) umfasst, wobei der Verriegelungsknopf (16.1) eingerichtet ist, um den Verriegelungsteil (14) in die Verriegelungskonfiguration zu verlagern, wobei der Entriegelungsknopf (16.2) eingerichtet ist, um den Verriegelungsteil (14) in die Entriegelungskonfiguration zu verlagern.

9. Zusätzlicher Griff (1) nach vorangehendem Anspruch, wobei der Verriegelungsknopf (16.1) und der Entriegelungsknopf (16.2) fest verbunden sind und sich auf zwei gegenüberliegenden Seiten der länglichen Aufnahme (10) befinden.

10. Zusätzlicher Griff (1) nach vorangehendem Anspruch, wobei die maximale Anzahl zwischen 5 und 50, beispielsweise zwischen 5 und 20, liegt.

11. Zusätzlicher Griff (1) nach einem der vorangehenden Ansprüche, wobei der Arretierungsteil (18) ausgelegt ist, um bei jeder Arretierung einer plastischen Verformung beispielsweise durch Verstemmen unterzogen zu werden, so dass der Arretierungsteil (18) verschlissen ist, wenn die maximale Anzahl erreicht ist.

12. Zusätzlicher Griff (1) nach vorangehendem Anspruch, wobei der Verriegelungsteil (14) eine Basis (14.2) hat, wobei der Arretierungsteil (18) mindestens eine Materialverstärkung umfasst, die sich auf einer jeweiligen Seite der Basis (14.2) erstreckt.

13. Zusätzlicher Griff (1) nach einem der vorangehenden Ansprüche, wobei der Empfangsteil (8) einen primären Durchgang (21) hat und der Greifteil (6) einen sekundären Durchgang (22) hat, wobei der primäre Durchgang (21) und der sekundäre Durchgang (22) übereinstimmen, so dass eine Führungsspindel durch den primären (21) und sekundären (22) Durchgang hindurchgehen kann.

14. Chirurgisches Instrumentenset (100), umfassend:
- einen zusätzlichen Griff (1) nach einem der vorangehenden Ansprüche, und
- mehrere chirurgische Instrumente (2), die jeweils einen Schaft (4) aufweisen, wobei die Außenfläche jedes Schafts (4) eine Nut (4.1) hat, die zur Aufnahme des Verriegelungsteils (14) geeignet ist, so dass die chirurgischen Instrumente (2) untereinander austauschbar sind.

15. Chirurgisches Instrumentenset (100) nach vorangehendem Anspruch, wobei die Außenfläche des Greifteils (6) Längsrillen (6.2) hat, wobei jede Längsrille (6.2) zur Aufnahme des Schafts (4) eines jeweiligen chirurgischen Instruments (2) derart geeignet ist, dass das chirurgische Instrument (2) durch elastisches Rasten befestigt ist.

## Claims

1. An ancillary handle (1) for manipulating a surgical instrument (2) including a rod (4), the ancillary handle (1) comprising:
- a gripping portion (6) configured to allow an operator to manipulate the ancillary handle (1),
- a receiving portion (8) having an elongated housing (10) configured to house at least partially the rod (4), and
- a locking device (12) configured to quickly lock the rod (4) in the elongated housing (10) so as to fasten the surgical instrument (2) to the ancillary handle (1);
the locking device (12) comprising a locking portion (14) configured to penetrate in a groove (4.1) located on the outer surface of the rod (4), the locking portion (14) being displaceable according to a direction of translation (Z14) with respect to the receiving portion (8) and between:
i) a locking configuration, in which the locking portion (14) can penetrate in the groove (4.1), so that the locking device (12) can block the surgical instrument (2) in the elongated housing (10), and
ii) an unlocking configuration, in which the locking portion (14) can exit the groove (4.1), so that the locking portion (14) can release the rod (4),
the locking device (12) further comprising an actuating portion (16) arranged to displace the locking portion (14) between the locking configuration and the unlocking configuration, the actuating portion (16) being secured to the locking portion (14).
the locking device (12) being **characterized in that** it includes at least one immobilizing portion (18), the immobilizing portion (18) being secured in translation to the locking portion (14), so that the immobilizing portion (18) is displaceable between:
i) a state of immobilization, in which the immobilizing portion (18) allow an immobilization of the locking device (12) with respect to the receiving portion (8) when the locking portion (14) is in a locking configuration, and
ii) a state of disengagement, in which the immobilizing portion (18) releases the locking device (12) of the receiving portion (8) when the locking portion (14) is in an unlocking configuration,
the immobilizing portion (18) being configured to authorize a maximum number of immobilizations, so that the immobilizing portion (18) no longer allows an immobilization when the maximum number is reached.

2. The ancillary handle (1) according to claim 1, wherein the elongated housing (10) has a shape comprising a cylinder angular section (10.1) and a flat part (10.2), the flat part (10.2) being suitable for cooperating with a complementary flat part (4.2) located on the outer surface of the rod (4), so as to prevent a rotation of the rod (4) in the elongated housing (10).

3. The ancillary handle (1) according to any one of the preceding claims, wherein the direction of translation (Z14) extends in a plane perpendicular (P14) to the direction of elongation (X10) of the elongated housing (10).

4. The ancillary handle (1) according to any one of the preceding claims, wherein the locking portion (14) is configured to have a displacement amplitude comprised between 2 mm and 8 mm.

5. The ancillary handle (1) according to any one of the preceding claims, wherein the locking portion (14) has a partially toroidal surface (14.1) so as to penetrate the groove (4.1) having a complementary partial toroidal shape.

6. The ancillary handle (1) according to the preceding claim, wherein the partially toroidal surface (14.1) extends over an angular sector (A14.1) comprised between 40 degrees and 180 degrees, the tip of the angular sector (A14.1) belonging to the axis of revolution of the partially toroidal surface (14.1).

7. The ancillary handle (1) according to any one of the preceding claims, wherein the actuating portion (16) and the receiving portion (8) are located at the same end of the gripping portion (6).

8. The ancillary handle (1) according to any one of the preceding claims, wherein the actuating portion (16) comprises a lock button (16.1) and an unlock button (16.2), the lock button (16.1) being arranged to displace the locking portion (14) towards the locking configuration, the unlock button (16.2) being arranged to displace the locking portion (14) towards the unlocking configuration.

9. The ancillary handle (1) according to the preceding claim, wherein the lock button (16.1) and the unlock button (16.2) are secured and located on two opposite sides of the elongated housing (10).

10. The ancillary handle (1) according to the preceding claim, wherein the maximum number is comprised between 5 and 50, for example between 5 and 20.

11. The ancillary handle (1) according to any one of the preceding claims, wherein the immobilizing portion (18) is configured to undergo a plastic deformation, for example by caulking, during each immobilization, so that the immobilizing portion (18) is worn out when the maximum number is reached.

12. The ancillary handle (1) according to the preceding claim, wherein the locking portion (14) has a base (14.2), the immobilizing portion (18) comprising at least an excess thickness of material which extends over a respective side of the base (14.2).

13. The ancillary handle (1) according to any one of the preceding claims, wherein the receiving portion (8) has a primary passage (21) and the gripping portion (6) has a secondary passage (22), the primary passage (21) and the secondary passage (22) being coincident so as to pass a guide pin through the primary (21) and secondary (22) passages.

14. A surgical instrument assembly (100) comprising:
- an ancillary handle (1) according to any one of the preceding claims, and
- a plurality of surgical instruments (2) each including a rod (4), the outer surface of each rod (4) having a groove (4.1) suitable to receive the locking portion (14), so that the surgical instruments (2) are interchangeable.

15. The surgical instrument assembly (100) according to the preceding claim, wherein the outer surface of the gripping portion (6) has longitudinal grooves (6.2), each longitudinal groove (6.2) being suitable to house the rod (4) of a respective surgical instrument (2) so as to fasten said surgical instrument (2) by elastic snap-fit.
